# EUROPEAN PATENT SPECIFICATION

(11) **EP 4 313 230 B1**
(45) Date of publication and mention of the grant of the patent: **19.02.2025**
(21) Application number: 22718757.2
(22) Date of filing: 14.03.2022
(51) Int. Cl.: A61M 16/04

(54) **TRACHEOSTOMY TUBES AND THEIR ASSEMBLY**
TRACHEOSTOMIETUBUS UND DESSEN ANORDNUNG
TUBES DE TRACHÉOTOMIE ET LEUR ASSEMBLAGE

(30) Priority: 26.03.2021 GB 202104357
(43) Date of publication of application: 07.02.2024
(73) Proprietor: ICU Medical International Limited, Lower Pemberton Ashford, Kent TN25 4BF (GB)
(72) Inventor: MORTON, Laura Beth, Kent TN15 8RS (GB); JEFFREY, Andrew Thomas, Kent TN29 0RB (GB); BATEMAN, Timothy, Kent TN29 0QD (GB); WOOSNAM, Christopher John, London SW12 8NY (GB)
(74) Representative: Flint, Jonathan McNeill
(86) International application number: PCT/GB2022/000031
(87) International publication number: WO 2022/200752

(56) References cited:
- EP-A1- 2 873 432
- WO-A1-2016/198817
- WO-A1-2021/209733
- DE-A1- 10 140 292
- US-A1- 2009 308 397
- US-A1- 2012 247 478
- US-A1- 2019 151 588
- US-B2- 8 469 024

## Description

This invention relates to tracheostomy tubes of the kind including a shaft with a mounting flange on the shaft.

Tracheal tubes are used to enable ventilation, respiration, or spontaneous breathing of a patient. Endotracheal tubes are inserted via the mouth or nose so that one end locates in the trachea and the other end locates outside the patient. Tracheostomy tubes are inserted into the trachea via a surgically formed opening in the neck. Tracheostomy tubes can be inserted by different techniques, such as the surgical cut-down procedure carried out in an operating theatre or a cricothyroidotomy procedure, which may be carried out in emergency situations.

Tracheostomy tubes are generally used for more long-term ventilation or where it is not possible to insert an airway through the mouth or nose. The patient is often conscious while breathing through a tracheostomy tube, which may be open to atmosphere or connected by tubing to some form of ventilator. The tube is secured in position on the patient's neck by means of a mounting flange fixed towards the machine end of the shaft of the tube and positioned to extend outwardly on opposite sides of the tube. A neck tie or the like is passed around the patient's neck and its ends are secured to either end of the flange. Alternatively, sutures can be used to secure the flange in place. DE10140292 describes a tracheostomy tube having a mounting flange and a detachable connector that can be fitted on the patient end of the tube. Each of WO2016/198817A1, US2019/151588A1 and US2009/308397A1 discloses a tracheostomy tube comprising a shaft and a flange mounted on the shaft, the flange having two radial arms for securing the tube to the neck of a patient, said flange being separately formed from the shaft.

Tracheostomy tubes can be made of various materials and are usually of a bendable plastics material such as PVC, polyurethane, or silicone. Silicone is particularly suitable because of the softness, comfort, and conformability the material provides. This is a particular advantage in tracheostomy tubes since the patient may be intubated and using a breathing machine for an extended time, usually more than one week. Silicone also has an advantage because it is not damaged by the high temperatures of an autoclave, thereby enabling a silicone tube to be cleaned and autoclaved for reuse.

It is particularly desirable for the mounting flange of a tracheostomy tube to be moulded from a soft, conformable plastics such as silicone so that it flexes readily to conform to the surface of the patient's neck. The flange may be moulded as an integral, single piece with the shaft of the tube. Alternatively, the flange may be moulded as a separate component from the shaft and subsequently assembled on and bonded to the shaft. This assembly and bonding process can be time consuming and lead to high manufacturing costs.

It is an object of the present invention to provide an alternative tracheostomy tube and a method of assembling such a tube.

According to one aspect of the present invention there is provided a tracheostomy tube of the above-specified kind, characterised in that the shaft includes a first retaining formation at the location of the flange, that the mounting flange is separately formed from the shaft and has two radial arms adapted to receive ends of a neck tie by which the tube is secured around the neck, and a retaining collar embracing the shaft, that the collar has a second retaining formation on an inner surface with which the first formation on the shaft is engaged such as to restrict longitudinal movement of the shaft relative to the collar, that the collar is deformable to allow it to be stretched outwardly to enable the first and second retaining formations to be engaged with one another, and that the shaft is secured with the flange by a bond between the outside of the shaft and the inside of the collar.

The first retaining formation on the shaft is preferably a projecting lug and the second retaining formation on the collar is preferably a recess. The shaft may have two retaining formations arranged diametrically opposite one another on the shaft, and the flange may have two retaining formations on the collar with which the respective retaining formations on the shaft are received. The shaft may additionally include one or more longitudinally extending alignment formations on its outer surface, the collar on the flange additionally including cooperating alignment formations on its inner surface. The machine end of the shaft is preferably provided with a connector, the retaining formation on the shaft being provided on a boss projecting outwardly of the shaft, and the boss being spaced from both the patient end of the shaft and the connector at the machine end of the shaft so that when the mounting flange is positioned against a patient's neck a length of shaft projects outwardly of the patient. The mounting flange is preferably of a deformable silicone material.

According to another aspect of the present invention there is provided a method of assembling a mounting flange on a shaft of a tracheostomy tube including the steps of providing a shaft including a first retaining formation at the desired location of the flange, providing a separately formed flange having a deformable retaining collar with a second cooperating retaining formation arranged to engage with the first retaining formation, applying a bonding substance to the outside of the shaft in the region of the first retaining formation or the inner surface of the collar or to both, threading the patient end of the shaft through the collar until contact with one of the retaining formations on the shaft prevents further insertion of the shaft in the collar, and deforming the collar outwardly to enable further insertion and to enable the first and second retaining formations to engage one another and thereby retain the flange on the shaft while the bonding substance cures.

According to a further aspect of the present invention there is provided a tracheostomy tube made according to the method of the above other aspect of the present invention.

A paediatric tracheostomy tube with a mounting flange and a method of assembly of the tube according to the present invention will now be described, by way of example, with reference to the accompanying drawings in which:
- Figure 1: is a perspective view of the tube;
- Figure 2: is a perspective view of the shaft of the tube before the mounting flange is assembled on the shaft;
- Figure 3: is a perspective view of the mounting flange before assembly on the shaft; and
- Figure 4: is a cross-sectional side elevation view of the tube in the region of the mounting flange.

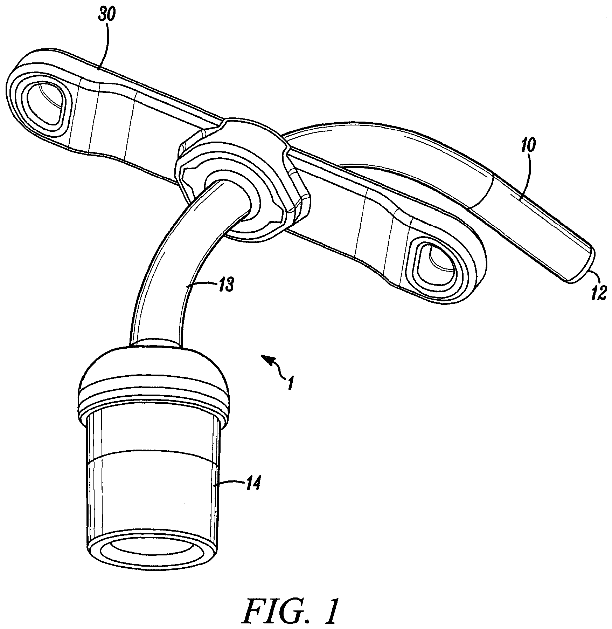

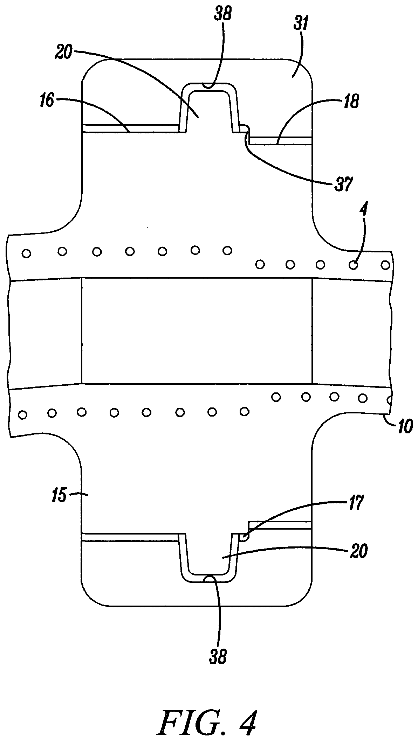

With reference first to Figure 1 the tracheostomy tube 1 has a curved shaft 10 of circular section moulded from a flexible plastics such as silicone and reinforced along its length by an embedded helix 4 (Figure 4) of a stiff material such as a metal or hard plastics. The patient, distal or forward end 12 of the tube 1 is adapted to locate in the trachea. The machine, proximal or rearward end 13 of the tube 1 extends externally of the patient and is terminated by a conventional externally tapered connector 14 forming a male coupling for mating with a cooperating female coupling (not shown) at the end of breathing tubing extending to a ventilator or the like. The shaft 10 extends through a tracheostomy opening in the neck of the patient and supports a mounting flange 30 about half way along its length by which the tube is secured with the patient's neck. This configuration leaves a length of the shaft 10 projecting externally from the mounting flange 30 as is common in paediatric tracheostomy tubes, to help isolate the connector 14 from the tracheostomy site. In alternative embodiments the flange could be positioned close to the machine end of the shaft, directly adjacent the connector.

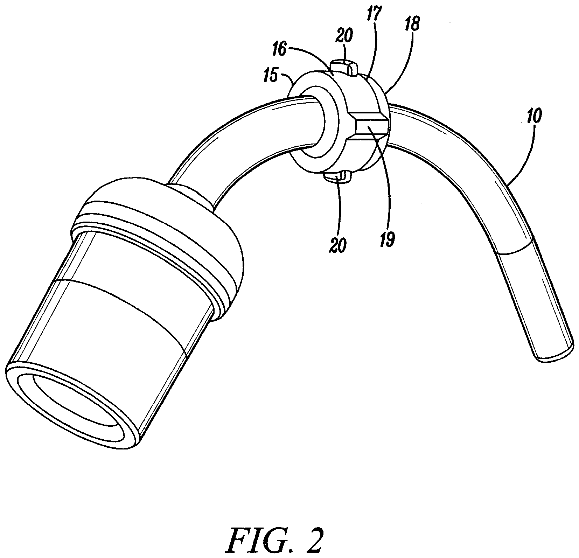

As most clearly shown in Figures 2 and 4, the shaft 10 includes an enlarged boss 15 moulded integrally with the shaft as a single piece and projecting radially externally of the shaft about midway along its length. The boss 15 has a generally cylindrical outer surface 16 of circular section with an annular step 17 to a short forward or patient end region 18 of reduced diameter. The external surface 16 of the boss 15 is interrupted by a pair of alignment formations or ribs 19 (only one of which is visible) positioned diametrically opposite one another. The alignment formations 19 extend longitudinally of the boss 15 from its forward end as far as the step 17 with the reduced diameter region 18. The alignment formations 19 present a rectangular shape at their outer end and taper slightly outwardly to a smaller width. The two alignment formations 19 are aligned along an axis extending orthogonally of the plane of curvature of the shaft 10. The boss 15 is completed by a first two mechanical retaining formations in the form of lugs 20 located close to and on the machine side of the step 17 with the reduced diameter region 18. The lugs 20 are rectangular and extend circumferentially by about 20°. The lugs 20 project radially outwardly by a distance substantially equal to or slightly less than the height of the alignment formations 19. The outer end of the lugs 20 are at a radial distance that exceeds the internal radius of the collar 31 at its rear or machine end.

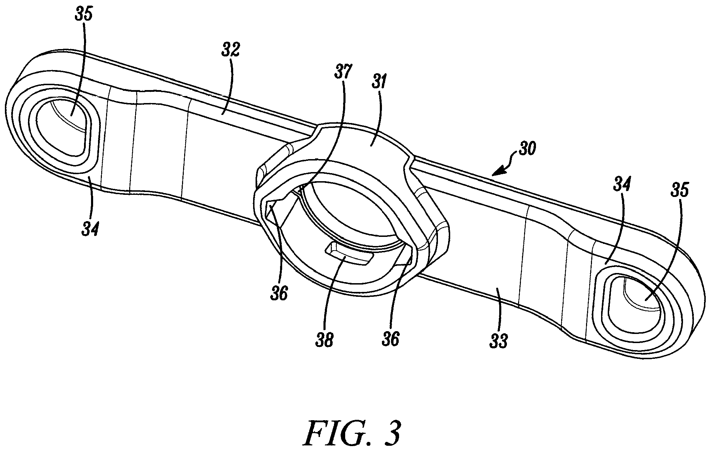

The mounting flange 30 is shown most clearly in Figure 3. The flange 30 comprises a unitary, single-piece moulding of a soft, deformable plastics such as silicone. The flange 30 may be of the same material as the shaft 10 or it may be of a different material or of a different grade of the same material. The flange 30 has a central circular collar 31 from which project two radial arms 32 and 33, diametrically opposite one another. The collar 31 is about three times the thickness of the main part of the arms 32 and 33 and projects rearwardly above the rear surface of the arms but lies level with the arms on their forward, patient side. At their outer ends the arms 32 and 33 have thickened regions 34 within each of which there is an opening 35 extending through the flange 30. The openings 35 receive the ends of a neck tie (not shown) or similar article by which the tube is secured about the neck of the patient. The collar 31 is moulded internally with two alignment slots 36 extending axially and tapered radially to a smaller width outwardly and having the same shape and size as the alignment formations 19 on the shaft 10. The slots 36 open at the rear end of the collar 31 and extend along the length of the collar as far as an internal annular step 37 approximately level with the rear surface of the arms 32 and 33. The collar 31 is thickened to accommodate the depth of the alignment slots 36 in the region of these slots. The collar 31 is also formed internally with a second two retaining formations in the form of recesses 38 of the same shape and size as, or slightly larger than, the retaining lugs 20 on the shaft 10. The recesses 38 are located diametrically opposite one another and midway between the two alignment slots 36. The retaining recesses 38 are located forwardly within the collar 31 adjacent the internal step 37. The shape, dimensions and positioning of the lugs 20 on the shaft 10 and the recesses 38 in the collar 31 are such that the lugs fit snuggly in the recesses when the step 17 on the boss 15 lies in contact with the step 37 in the collar. The shaft 10 is permanently secured with the mounting flange 30 by means of a bonding substance applied between the contacting surfaces on the outside of the boss 15 and the inside of the collar 31. The bonding substance could be of any conventional kind such as a solvent, adhesive or glue.

The tube 1 is shown without any sealing cuff but it could be provided with such a conventional sealing cuff and other conventional features such as vocalisation fenestrations or suction lumens.

The tube is assembled by first moulding the shaft 10 and flange 30 separately. A bonding substance is then applied to either the outside of the boss 15 on the shaft 10 or to the inside of the collar 31 on the flange 30 or to both the boss and collar. The patient end of the shaft 10 is then threaded through the collar 31 from its rear or machine side. The shaft 10 is advanced through the collar 31 until the forward end region 18 of the boss 15 locates in the rear end of the collar. The shaft 10 is twisted as necessary to bring the alignment formations 19 into alignment with the alignment slots 36 in the collar 31 and ensure the correct orientation of the shaft relative to the mounting flange 30. The two retaining lugs 20 now contact the rear end of the collar 31 and impede further free insertion. However, the deformable nature of the collar 31 enables it to be manipulated over the lugs 20 to allow the boss 15 to be inserted in the collar. When the lugs 20 come level with the retaining recesses 38, the collar 31 relaxes about the boss 15 with the lugs received within the recesses. The engaged lugs 20 and recesses 38 serve to prevent any inadvertent longitudinal movement of the shaft relative to the flange, thereby retaining the two components together mechanically while the bonding substance cures. It can be seen that the engagement of the lugs 20 in the recesses 38 also resists rotation between the components 10 and 30 during the bonding process. This means that the assembler does not need to hold the components 10 and 30 together while the bonding substance cures, can release the assembly, and go on to assemble the next tube.

The retaining formations on the shaft and collar need not be provided by a projection on the shaft and a recess on the collar but could, instead, be provided by a recess on the shaft and an engaging projection on the collar. The arrangement with the recess in the collar is preferred because it is easier to remove a flange with such a feature from its mould. The tube could have just one retaining formation on the shaft and collar, or more than the two described.

## Claims

1. A tracheostomy tube (1) including a shaft (10) and mounting flange (30) on the shaft, the mounting flange (30) being separately formed from the shaft (10) and having two radial arms (32 and 33) adapted to receive ends of a neck tie by which the tube is secured around the neck, and a retaining collar (31) embracing the shaft (10), **characterized in that**
the shaft (10) includes a first retaining formation (20) at the location of the flange (30), and
the collar (31) has a second retaining formation (38) on an inner surface with which the first formation (20) on the shaft (10) is engaged such as to restrict longitudinal movement of the shaft (10) relative to the collar (31), that the collar (31) is deformable to allow it to be stretched outwardly to enable the first and second retaining formations (20 and 38) to be engaged with one another, and that the shaft (10) is secured with the flange (30) by a bond between the outside of the shaft (10) and the inside of the collar (31).

2. A tracheostomy tube (1) according to Claim 1, **characterised in that** the first retaining formation on the shaft (10) is a projecting lug (20) and the second retaining formation on the collar (31) is a recess (38).

3. A tracheostomy tube (1) according to Claim 1 or 2, **characterised in that** the shaft (10) has two retaining formations (20) arranged diametrically opposite one another on the shaft, and that the flange (30) has two retaining formations (38) on the collar (31) with which the respective retaining formations (20) on the shaft (10) are received.

4. A tracheostomy tube according to any one of the preceding claims, **characterised in that** the shaft (10) additionally includes one or more longitudinally extending alignment formations (19) on its outer surface, and that the collar (31) on the flange (30) additionally includes cooperating alignment formations (36) on its inner surface.

5. A tracheostomy tube (1) according to any one of the preceding claims, **characterised in that** the machine end of the shaft (10) is provided with a connector (14), that the retaining formation (20) on the shaft is provided on a boss (15) projecting outwardly of the shaft, and that the boss (15) is spaced from both the patient end (12) of the shaft and the connector (14) at the machine end of the shaft so that when the mounting flange (30) is positioned against a patient's neck a length of shaft (10) projects outwardly of the patient.

6. A tracheostomy tube according to any one of the preceding claims wherein the mounting flange (30) is of a deformable silicone material.

7. A method of assembling a mounting flange (30) on a shaft (10) of a tracheostomy tube (1) including the steps of providing a shaft (10) including a first retaining formation (20) at the desired location of the flange (30), providing a separately formed flange (30) having a deformable retaining collar (31) with a second cooperating retaining formation (38) arranged to engage with the first retaining formation (20), applying a bonding substance to the outside of the shaft (10) in the region of the first retaining formation (20) or the inner surface of the collar (31) or to both, threading the patient end (12) of the shaft (10) through the collar (31) until contact with one of the retaining formations (20) on the shaft prevents further insertion of the shaft in the collar, and deforming the collar (31) outwardly to enable further insertion and to enable the first and second retaining formations (20 and 38) to engage one another and thereby retain the flange (30) on the shaft (10) while the bonding substance cures.

8. A method according to Claim 7, **characterised in that** the shaft (10) additionally includes one or more longitudinally extending alignment formations (19) on its outer surface, that the collar (31) includes cooperating alignment formations (36) on its inner surface, and that the assembly method additionally including the step of orienting the mounting flange (30) with respect to the shaft (10) such that the alignment formations (19 and 36) align with each other before the collar (31) is slid into a retaining position.

9. A tracheostomy tube including a mounting flange (30) assembled on the shaft (10) of a tube by a method according to Claim 7 or 8.

## Patentansprüche

1. Tracheostomietubus (1) mit einem Schaft (10) und einem Befestigungsflansch (30) an dem Schaft, wobei der Befestigungsflansch (30) getrennt vom Schaft (10) ausgebildet ist und zwei radiale Arme (32 und 33) aufweist, die zur Aufnahme von Enden einer Halsbinde geeignet sind, mit welcher der Tubus um den Hals befestigt wird, und mit einem Haltekragen (31), der den Schaft (10) umschließt, **dadurch gekennzeichnet, dass** der Schaft (10) eine erste Halteformation (20) an der Stelle des Flanschs (30) aufweist, und der Kragen (31) eine zweite Halteformation (38) an einer Innenfläche aufweist, mit der die erste Formation (20) mit dem Schaft (10) in Eingriff steht, um eine Längsbewegung des Schafts (10) relativ zum Kragen (31) zu begrenzen, dass der Kragen (31) verformbar ist, so dass er nach außen gedehnt werden kann, um das Ineinandergreifen der ersten und der zweiten Halteformation (20 und 38) zu ermöglichen, und dass der Schaft (10) an dem Flansch (30) durch eine Klebeverbindung zwischen der Außenseite des Schafts (10) und der Innenseite des Kragens (31) befestigt ist.

2. Tracheostomietubus (1) nach Anspruch 1, **dadurch gekennzeichnet, dass** die erste Halteformation am Schaft (10) eine vorstehende Nase (20) und die zweite Halteformation am Kragen (31) eine Ausnehmung (38) ist.

3. Tracheostomietubus (1) nach Anspruch 1 oder 2, **dadurch gekennzeichnet, dass** der Schaft (10) zwei diametral gegenüberliegend am Schaft angeordnete Halteformationen (20) aufweist, und dass der Flansch (30) zwei Halteformationen (38) am Kragen (31) aufweist, mit denen die jeweiligen Halteformationen (20) am Schaft (10) aufgenommen werden.

4. Tracheostomietubus nach einem der vorhergehenden Ansprüche, **dadurch gekennzeichnet, dass** der Schaft (10) an seiner Außenfläche zusätzlich eine oder mehrere längs verlaufende Ausrichtungsformationen (19) aufweist und dass der Kragen (31) am Flansch (30) an seiner Innenfläche zusätzlich zusammenwirkende Ausrichtungsformationen (36) aufweist.

5. Tracheostomietubus (1) nach einem der vorhergehenden Ansprüche, **dadurch gekennzeichnet, dass** das Maschinenende des Schafts (10) mit einem Anschlussstück (14) versehen ist, dass die Halteformation (20) an dem Schaft an einem vom Schaft nach außen vorstehenden Vorsprung (15) vorgesehen ist, und dass der Vorsprung (15) sowohl zum Patientenende (12) des Schafts als auch zum Verbindungsstück (14) am Maschinenende des Schafts beabstandet ist, so dass, wenn der Befestigungsflansch (30) am Hals eines Patienten positioniert ist, ein Stück des Schafts (10) vom Patienten weg ragt.

6. Tracheostomietubus nach einem der vorhergehenden Ansprüche, wobei der Befestigungsflansch (30) aus einem verformbaren Silikonmaterial besteht.

7. Verfahren zur Montage eines Befestigungsflansches (30) an einem Schaft (10) eines Tracheostomietubus (1), umfassend die folgenden Schritte: Bereitstellen eines Schafts (10) mit einer ersten Halteformation (20) an der gewünschten Position des Flansches (30), Bereitstellen eines separat geformten Flansches (30) mit einem verformbaren Haltekragen (31) mit einer zweiten zusammenwirkenden Halteformation (38), die so angeordnet ist, dass sie mit der ersten Halteformation (20) in Eingriff kommt, Aufbringen einer Klebesubstanz auf die Außenseite des Schafts (10) im Bereich der ersten Halteformation (20) oder auf die Innenfläche des Kragens (31) oder auf beide, Einfädeln des Patientenendes (12) des Schafts (10) durch den Kragen (31), bis der Kontakt mit einer der Halteformationen (20) am Schaft ein weiteres Einführen des Schafts in den Kragen verhindert, und Verformen des Kragens (31) nach außen, um ein weiteres Einführen zu ermöglichen und um es zu ermöglichen, dass die erste und die zweite Halteformation (20 und 38) ineinander eingreifen und dadurch den Flansch (30) an dem Schaft (10) festhalten, während die Klebesubstanz aushärtet.

8. Verfahren nach Anspruch 7, **dadurch gekennzeichnet, dass** der Schaft (10) zusätzlich eine oder mehrere sich in Längsrichtung erstreckende Ausrichtungsformationen (19) an seiner Außenfläche aufweist, dass der Kragen (31) zusammenwirkende Ausrichtungsformationen (36) an seiner Innenfläche aufweist, und dass das Montageverfahren zusätzlich den Schritt des Ausrichtens des Befestigungsflansches (30) in Bezug auf den Schaft (10) umfasst, so dass die Ausrichtungsformationen (19 und 36) miteinander fluchten, bevor der Kragen (31) in eine Halteposition geschoben wird.

9. Tracheostomietubus mit einem Befestigungsflansch (30), der nach einem Verfahren nach Anspruch 7 oder 8 am Schaft (10) eines Tubus montiert wird.

## Revendications

1. Tube de trachéotomie (1) comprenant une tige (10) et une bride de montage (30) sur la tige, la bride de montage (30) étant formée séparément de la tige (10) et comportant deux bras radiaux (32 et 33) adaptés pour recevoir les extrémités d'un collier de serrage par lequel le tube est fixé autour du cou, et un collier de retenue (31) englobant la tige (10), **caractérisé en ce que** la tige (10) comprend une première formation de retenue (20) à l'emplacement de la bride (30), et le collier (31) comporte une seconde formation de retenue (38) sur une surface intérieure avec laquelle la première formation (20) sur la tige (10) est engagée de manière à restreindre le mouvement longitudinal de la tige (10) par rapport au collier (31), que le collier (31) est déformable pour lui permettre d'être étiré vers l'extérieur pour permettre aux première et seconde formations de retenue (20 et 38) d'être engagées l'une avec l'autre, et que la tige (10) est fixée à la bride (30) par une liaison entre l'extérieur de la tige (10) et l'intérieur du collier (31).

2. Tube de trachéotomie (1) selon la revendication 1, **caractérisé en ce que** la première formation de retenue sur la tige (10) est un ergot en saillie (20) et la seconde formation de retenue sur le collier (31) est un évidement (38).

3. Tube de trachéotomie (1) selon la revendication 1 ou 2, **caractérisé en ce que** la tige (10) comporte deux formations de retenue (20) disposées diamétralement opposées l'une à l'autre sur la tige, et **en ce que** la bride (30) comporte deux formations de retenue (38) sur le collier (31) avec lesquelles les formations de retenue respectives (20) sur la tige (10) sont reçues.

4. Tube de trachéotomie (1) selon l'une quelconque des revendications précédentes, **caractérisé en ce que** la tige (10) comprend en outre une ou plusieurs formations d'alignement (19) s'étendant longitudinalement sur sa surface extérieure, et **en ce que** le collier (31) sur la bride (30) comprend en outre des formations d'alignement coopérantes (36) sur sa surface intérieure.

5. Tube de trachéotomie (1) selon l'une quelconque des revendications précédentes, **caractérisé en ce que** l'extrémité machine de la tige (10) est pourvue d'un connecteur (14), que la formation de retenue (20) sur la tige est pourvue d'un bossage (15) faisant saillie vers l'extérieur de la tige, et que le bossage (15) est espacé à la fois de l'extrémité patient (12) de la tige et du connecteur (14) à l'extrémité machine de la tige, de sorte que lorsque la bride de montage (30) est positionnée contre le cou du patient, une longueur de la tige (10) fait saillie vers l'extérieur du patient.

6. Tube de trachéotomie selon l'une quelconque des revendications précédentes, dans lequel la bride de montage (30) est en un matériau silicone déformable.

7. Procédé d'assemblage d'une bride de montage (30) sur une tige (10) d'un tube de trachéotomie (1), comprenant les étapes consistant à fournir une tige (10) comprenant une première formation de retenue (20) à l'emplacement souhaité de la bride (30), fournir une bride formée séparément (30) ayant un collier de retenue déformable (31) avec une seconde formation de retenue coopérante (38) agencée pour s'engager avec la première formation de retenue (20), appliquer une substance de liaison sur l'extérieur de la tige (10) dans la région de la première formation de retenue (20) ou sur la surface intérieure du collier (31) ou sur les deux, enfiler l'extrémité patient (12) de la tige (10) à travers le collier (31) jusqu'à ce qu'un contact avec l'une des formations de retenue (20) sur la tige empêche une insertion ultérieure de la tige dans le collier, et déformer le collier (31) vers l'extérieur pour permettre une insertion ultérieure et pour permettre aux première et secondes formations de retenue (20 et 38) de s'engager l'une dans l'autre et ainsi retenir la bride (30) sur la tige (10) pendant que la substance de liaison durcit.

8. Procédé selon la revendication 7, **caractérisé en ce que** la tige (10) comprend en outre une ou plusieurs formations d'alignement (19) s'étendant longitudinalement sur sa surface extérieure, **en ce que** le collier (31) comprend des formations d'alignement coopérantes (36) sur sa surface intérieure, et que le procédé d'assemblage comprend en outre l'étape consistant à orienter la bride de montage (30) par rapport à la tige (10) de sorte que les formations d'alignement (19 et 36) s'alignent l'une avec l'autre avant que le collier (31) ne soit glissé dans une position de maintien.

9. Tube de trachéotomie comprenant une bride de montage (30) assemblée sur la tige (10) d'un tube par un procédé selon la revendication 7 ou 8.
